# EUROPEAN PATENT SPECIFICATION

(11) **EP 2 575 898 B1**
(45) Date of publication and mention of the grant of the patent: **30.09.2020**
(21) Application number: 11722429.5
(22) Date of filing: 30.05.2011
(51) Int. Cl.: A61K 51/04, A61K 101/02, C07B 59/00, C07C 213/08

(54) **METHOD FOR PRODUCTION OF F-18 LABELED AMYLOID BETA LIGAND**
VERFAHREN ZUR HERSTELLUNG VON F-18-MARKIERTEN AMLYLOID-BETA-LIGANDEN
PROCÉDÉ DE PRODUCTION DE LIGAND DE L'AMYLOÏDE BÊTA MARQUÉ AU 18F

(30) Priority: 04.06.2010 EP 10164948
(43) Date of publication of application: 10.04.2013
(73) Proprietor: Life Molecular Imaging SA, 1753 Matran (CH)
(72) Inventor: BERNDT, Mathias, 12163 Berlin (DE); FRIEBE, Matthias, 13509 Berlin (DE); HULTSCH, Christina, 12101 Berlin (DE); SAMSON, Fabrice, Seoul 140-858 (KR); OH, Seung, Jun, Seoul 150-045 (KR); SMUDA, Christoph, CH-8952 Schlieren (CH)
(74) Representative: Kilger, Ute
(86) International application number: PCT/EP2011/058819
(87) International publication number: WO 2011/151282

(56) References cited:
- WO-A1-2010/078370
- WO-A1-2011/003591
- WO-A2-2010/000409
- US-A1- 2006 269 473
- US-A1- 2010 113 763
- ZHANG ET AL: "F-18 Polyethyleneglycol stilbenes as PET imaging agents targeting Abeta aggregates in the brain", NUCLEAR MEDICINE AND BIOLOGY, ELSEVIER, NY, US, vol. 32, no. 8, 1 November 2005 (2005-11-01), pages 799-809, XP005129336, ISSN: 0969-8051, DOI: 10.1016/J.NUCMEDBIO.2005.06.001 cited in the application
- WANG H ET AL: "Facile and rapid one-step radiosynthesis of [<18>F]BAY94-9172 with a new precursor", NUCLEAR MEDICINE AND BIOLOGY, ELSEVIER, NY, US, vol. 38, no. 1, 1 January 2011 (2011-01-01), pages 121-127, XP027589497, ISSN: 0969-8051 [retrieved on 2010-08-16] cited in the application
- PATT M ET AL: "Metabolite analysis of [18F]Florbetaben (BAY 94-9172) in human subjects: a substudy within a proof of mechanism clinical trial", JOURNAL OF RADIOANALYTICAL AND NUCLEAR CHEMISTRY, KLUWER ACADEMIC PUBLISHERS, DO, vol. 284, no. 3, 16 March 2010 (2010-03-16), pages 557-562, XP019792449, ISSN: 1588-2780
- LIN K J ET AL: "Whole-body biodistribution and brain PET imaging with [<18>F]AV-45, a novel amyloid imaging agent - a pilot study", NUCLEAR MEDICINE AND BIOLOGY, ELSEVIER, NY, US, vol. 37, no. 4, 1 May 2010 (2010-05-01), pages 497-508, XP027039103, ISSN: 0969-8051 [retrieved on 2010-04-07]
- LIU Y ET AL: "Optimization of automated radiosynthesis of [<18>F]AV-45: a new PET imaging agent for Alzheimer's disease", NUCLEAR MEDICINE AND BIOLOGY, ELSEVIER, NY, US, vol. 37, no. 8, 1 November 2010 (2010-11-01), pages 917-925, XP027472064, ISSN: 0969-8051, DOI: 10.1016/J.NUCMEDBIO.2010.05.001 [retrieved on 2010-06-29] cited in the application
- YAO C H ET AL: "GMP-compliant automated synthesis of [<18>F]AV-45 (Florbetapir F 18) for imaging beta-amyloid plaques in human brain", APPLIED RADIATION AND ISOTOPES, ELSEVIER, OXFORD, GB, vol. 68, no. 12, 1 December 2010 (2010-12-01), pages 2293-2297, XP027265706, ISSN: 0969-8043 [retrieved on 2010-07-07] cited in the application
- PIYUSH KUMAR ET AL: "18F-FESB: synthesis and automated radiofluorination of a novel18F-labeled pet tracer for[beta]-amyloid plaques", JOURNAL OF LABELLED COMPOUNDS AND RADIOPHARMACEUTICALS, vol. 48, no. 13, 1 November 2005 (2005-11-01), pages 983-996, XP55006268, ISSN: 0362-4803, DOI: 10.1002/jlcr.1011

## Description

### FIELD OF INVENTION

This invention relates to methods, which provide access to [F-18]fluoropegylated (aryl/heteraryl vinyl)-phenyl methyl amine derivatives.

### BACKGROUND

Alzheimer's Disease (AD) is a progressive neurodegenerative disorder marked by loss of memory, cognition, and behavioral stability. AD is defined pathologically by extracellular senile plaques comprised of fibrillar deposits of the beta-amyloid peptide (Aβ) and neurofibrillary tangles comprised of paired helical filaments of hyperphosphorylated tau. The 39 - 43 amino acids comprising Aβ peptides are derived from the larger amyloid precursor protein (APP). In the amyloidogenic pathway, Aβ peptides are cleaved from APP by the sequential proteolysis by beta- and gamma-secretases. Aβ peptides are released as soluble proteins and are detected at low level in the cerebrospinal fluid (CSF) in normal aging brain. During the progress of AD the Aβ peptides aggregate and form amyloid deposits in the parenchyma and vasculature of the brain, which can be detected post mortem as diffuse and senile plaques and vascular amyloid during histological examination (for a recent review see: Blennow et al. Lancet. 2006 Jul 29;368(9533):387-403).
Alzheimers disease (AD) is becoming a great health and social economical problem all over the world. There are great efforts to develop techniques and methods for the early detection and effective treatment of the disease. Currently, diagnosis of AD in an academic memory-disorders clinic setting is approximately 85-90% accurate (Petrella JR et al. Radiology. 2003 226:315-36). It is based on the exclusion of a variety of diseases causing similar symptoms and the careful neurological and psychiatric examination, as well as neuropsychological testing.

Molecular imaging has the potential to detect disease progression or therapeutic effectiveness earlier than most conventional methods in the fields of neurology, oncology and cardiology. Among the several promising molecular imaging technologies, such as optical imaging, MRI, SPECT and PET, PET is of particular interest for drug development because of its high sensitivity and ability to provide quantitative and kinetic data.
For example positron emitting isotopes include e.g. carbon, iodine, nitrogen, and oxygen. These isotopes can replace their non-radioactive counterparts in target compounds to produce PET tracers that have similar biological properties. Among these isotopes F-18 is a preferred labeling isotope due to its half life of 110 min, which permits the preparation of diagnostic tracers and subsequent study of biochemical processes. In addition, its low β+ energy (634 keV) is also advantageous.

Post-mortem histological examination of the brain is still the only definite diagnosis of Alzheimer's disease. Thus, the *in vivo* detection of one pathological feature of the disease - the amyloid aggregate deposition in the brain - is thought to have a strong impact on the early detection of AD and differentiating it from other forms of dementia. Additionally, most disease modifying therapies which are in development are aiming at lowering of the amyloid load in the brain. Thus, imaging the amyloid load in the brain may provide an essential tool for patient stratification and treatment monitoring (for a recent review see: Nordberg. Eur J Nucl Med Mol Imaging. 2008 Mar;35 Suppl 1:S46-50).
In addition, amyloid deposits are also known to play a role in amyloidoses, in which amyloid proteins (e.g. tau) are abnormally deposited in different organs and/or tissues, causing disease. For a recent review see Chiti et al. Annu Rev Biochem. 2006;75:333-66.

Since nucleophilc radiofluorination was performed using nano- and subnanomolar quantities of [F-18]fluoride is it well known, that only small amounts of labeling precursor are necessary for successful radiolabeling. Generally, few micromole of precursor provide a huge excess with respect to the radioisotope, resulting in pseudo-first-order reaction kinetics (P. W. Miller et al., Ang. Chem. Int. Ed. 47 (2008) 8998-9033).

Direct radiofluorinations of fluoropegylated bis-aryl/heteroaryl Aβ ligands L have been described in the literature.

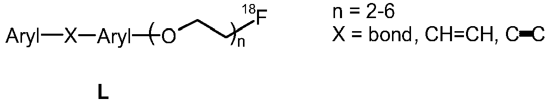

### a) Diphenylacetylenes B

### (R. Chandra et al. J. Med Chem. 50 (2007) 2415-2423)

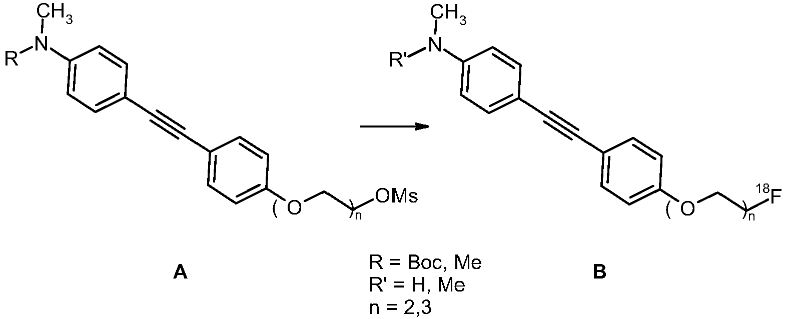

Labelings of 1 mg (1.83-2.48 µmol) precursor **A** in DMSO using potassium carbonate/kryptofix complex afforded **B** in 20-30% radiochemical yield (decay corrected).

### b) Indolinyl- and indolylphenylacetylenes D

### (R. Chandra et al. J. Med Chem. 50 (2007) 2415-2423)

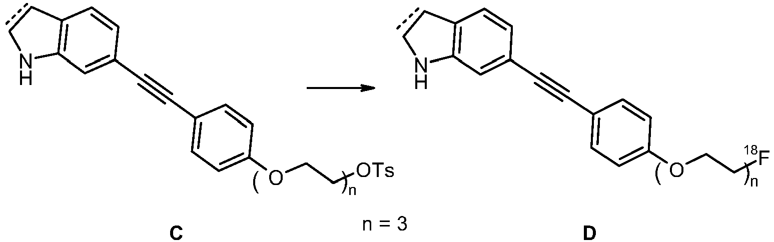

Labelings of precursor **C** in DMSO using potassium carbonate/kryptofix complex afforded **D** in 11-16% radiochemical yield (decay corrected).

### c) Flavone derivatives F

### (M. Ono et al. Bioorg. Med. Chem. 17 (2009) 2069-2076)

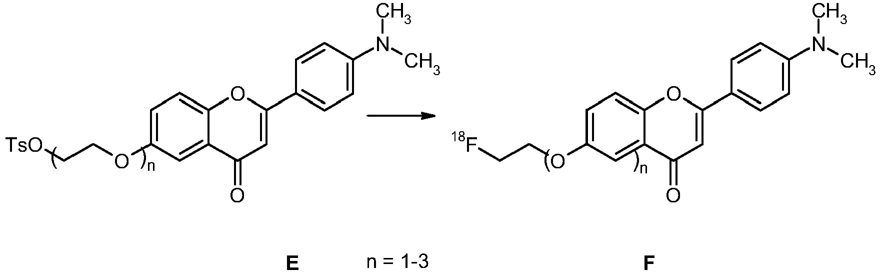

Labelings of 0.2 mg (0.35-0.42 µmol) precursor **E** in DMSO using potassium carbonate/kryptofix complex afforded **F** in 5-13% radiochemical yield (decay corrected).

### d) Phen-Naphthalene and Phen-quinoline derivatives H

### (WO2008124812)

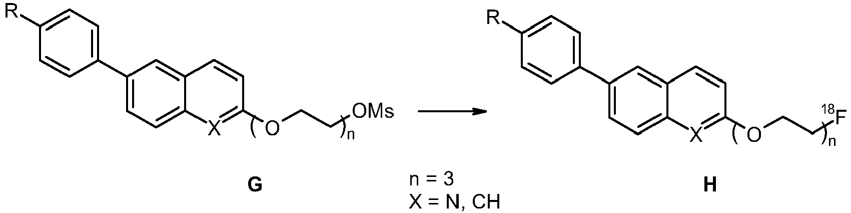

Labelings of 1 mg (≤ 2.11 µmol) precursor **G** in DMSO using potassium carbonate/kryptofix complex afforded **H** in 30% radiochemical yield (decay corrected).

### e) Benzothiazole derivatives J

### (WO2007002540)

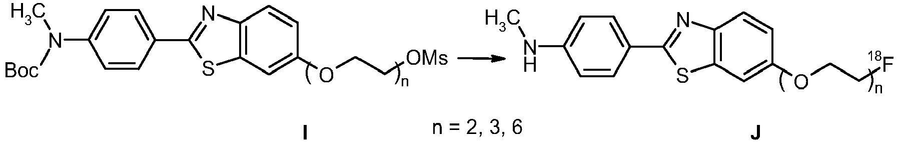

Labelings of precursor **I** in DMSO using potassium carbonate/kryptofix complex afforded **H** in 11-35% radiochemical yield (decay corrected).
The influence of amount of precursor was investigated (example n = 2), and 1-3 mg (1.91-5.74µmol) were found to be optimal for this kind of conversion.

Also fluoropegylated (aryl/heteraryl vinyl)-phenyl methyl amines such as 4-[(E)-2-(4-{2-[2-(2-fluoroethoxy)ethoxy]ethoxy}phenyl)vinyl]-N-methylaniline and 4-[(E)-2-(6-{2-[2-(2-fluoroethoxy)ethoxy]ethoxy}pyridin-3-yl)vinyl]-N-methylaniline have been labeled with F-18 fluoride before and are covered by patent applications WO2006066104, WO2007126733 and members of the corresponding patent families.

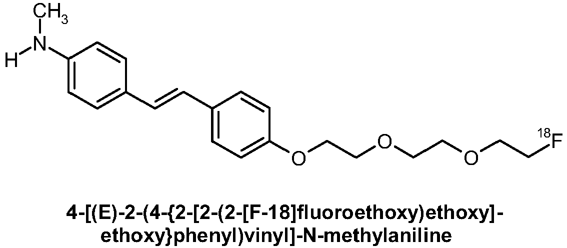

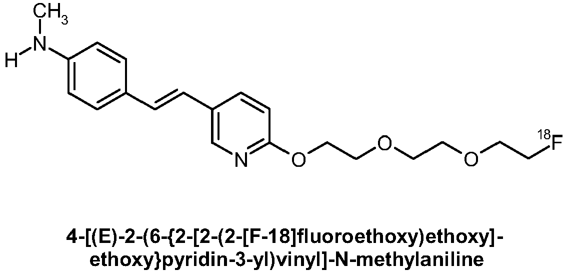

### 4-[(E)-2-(4-{2-[2-(2-[F-18]fluoroethoxy)ethoxy]ethoxy}phenyl)vinyl]-N-methylaniline

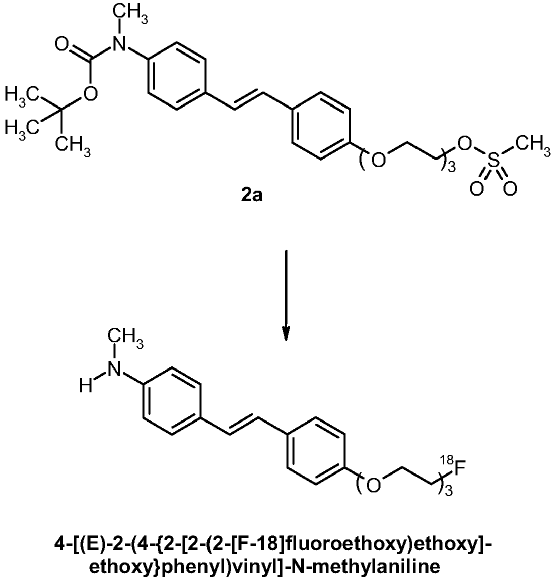

### a) W. Zhang et al., Nuclear Medicine and Biology 32 (2005) 799-809

4 mg (7.47 µmol) precursor **2a** (2-[2-(2-{4-[(E)-2-{4-[(tert-butoxycarbonyl)(methyl)amino]-phenyl}vinyl]phenoxy}ethoxy)ethoxy]ethyl methanesulfonate) in 0.2 mL DMSO were reacted with [F-18]fluoride/kryptofix/potassium carbonate complex. The intermediate was deprotected with HCl and neutralized with NaOH. The mixture was extracted with ethyl acetate. The solvent was dried and evaporated. The residue was dissolved in acetonitrile and purified by semi-preparative HPLC. 20% (decay corrected), 11% (not corrected for decay) 4-[(E)-2-(4-{2-[2-(2-[F-18]fluoroethoxy)ethoxy]ethoxy}phenyl)vinyl]-N-methylaniline were obtained within 90 min.

### b) WO2006066104

4 mg (7.47 µmol) precursor **2a** (2-[2-(2-{4-[(E)-2-{4-[(tert-butoxycarbonyl)(methyl)amino]-phenyl}vinyl]phenoxy}ethoxy)ethoxy]ethyl methanesulfonate) in 0.2 mL DMSO were reacted with [F-18]fluoride/kryptofix/potassium carbonate complex. The intermediate was deprotected with HCl and neutralized with NaOH. The mixture was extracted with ethyl acetate. The solvent was dried and evaporated, the residue was dissolved in acetonitrile and purified by semi-preparative HPLC. 30% (decay corrected), 17% (not corrected for decay) 4-[(E)-2-(4-{2-[2-(2-[F-18]fluoroethoxy)ethoxy]ethoxy}phenyl)vinyl]-N-methylaniline were obtained in 90 min.

Very recently, further syntheses have been described:
a) US20100113763
   **2a** (2-[2-(2-{4-[(E)-2-{4-[(tert-butoxycarbonyl)(methyl)amino]phenyl}vinyl]-phenoxy}ethoxy)ethoxy]ethyl methanesulfonate) was reacted with [F-18]fluoride reagent in a mixture of 500 µL *tert*-alcohol and 100 µL acetonitrile. After fluorination, the solvent was evaporated and a mixture of HCl and acetonitrile was added. After deprotection (heating at 100-120°C), the crude product mixture was purified by HPLC (C18, 60% acetonitrile, 40% 0.1M ammonium formate). It is outlined, that at a low level of [F-18]fluoride activity (4.1 mCi = 0.15 GBq) similar yields are obtained with 2-10 mg precursor. In contrast to the data published, we found that at higher levels of [F-18]fluoride activity (e.g. 30-55 GBq) yields strongly depend on the amount of precursor (Example 2). Additionally and in contrast to the published data, the yields given in Example 2 refer to a product suitable for human use, obtained by a full manufacturing processes.
b) H. Wang et al., Nuclear Medicine and Biology 38 (2011) 121-127
   5 mg (9.33 µmol) precursor **2a** (2-[2-(2-{4-[(E)-2-{4-[(tert-butoxycarbonyl)(methyl)amino]-phenyl}vinyl]phenoxy}ethoxy)ethoxy]ethyl methanesulfonate) in 0.5 mL DMSO were reacted with [F-18]fluoride/kryptofix/potassium carbonate complex. The intermediate was deprotected with HCl and neutralized with NaOH. The crude product was diluted with acetonitrile / 0.1M ammonium formate (6/4) and purified by semi-preparative HPLC. The product fraction was collected, diluted with water, passed through a C18 cartridge and eluted with ethanol, yielding 17% (not corrected for decay) 4-[(E)-2-(4-{2-[2-(2-[F-18]fluoroethoxy)ethoxy]ethoxy}phenyl)vinyl]-N-methylaniline within 50 min. In the publication, the conversion of an unprotected mesylate precursor (is described:
   5 mg (11.48 µmol) unprotected mesylate precursor (2-{2-[2-(4-{(E)-2-[4-(methylamino)phenyl]vinyl}phenoxy)ethoxy]ethoxy}ethyl 4-methanesulfonate) in 0.5 mL DMSO were reacted with [F-18]fluoride/kryptofix/potassium carbonate complex. The crude product was diluted with acetonitrile / 0.1M ammonium formate (6/4) and purified by semi-preparative HPLC. The product fraction was collected, diluted with water, passed through a C18 cartridge and eluted with ethanol, yielding 23% (not corrected for decay) 4-[(E)-2-(4-{2-[2-(2-[F-18]fluoroethoxy)ethoxy]ethoxy}phenyl)vinyl]-N-methylaniline within 30 min.

### 4-[(E)-2-(6-{2-[2-(2-[F-18]fluoroethoxy)ethoxy]ethoxy}pyridin-3-yl)vinyl]-N-methylaniline

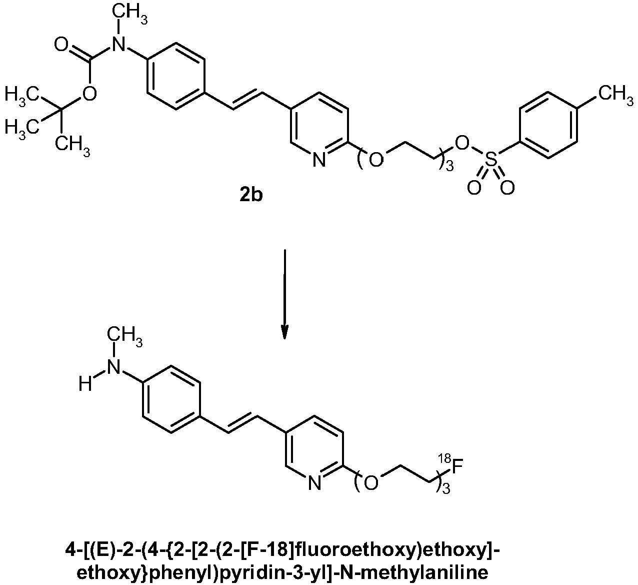

### a) S. R. Choi et al., The Journal of Nuclear Medicine 50 (2009) 1887-1894.

1 mg precursor (1.63 µmol) **2b** (2-{2-[2-({5-[(E)-2-{4-[(tert-butoxycarbonyl)(methyl)amino]-phenyl}vinyl]pyridin-2-yl}oxy)ethoxy]ethoxy}ethyl 4-methylbenzenesulfonate) in 1 mL DMSO was reacted with [F-18]fluoride/kryptofix/potassium carbonate complex. The intermediate was deprotected with HCl and neutralized with NaOH. DMSO and inorganic components were removed by solid-phase-extraction on SepPak light C18 cartridge (Waters). The crude product was purified by semi-preparative HPLC. The product fraction was diluted with water and passed through a SepPak light C18 cartridge. The radiotracer was eluted with ethanol. The yield for 4-[(E)-2-(6-{2-[2-(2-[F-18]fluoroethoxy)ethoxy]ethoxy}pyridin-3-yl)vinyl]-N-methylaniline was 10-30% (decay corrected).

### b) WO2010078370

1.5 mg (2.45 µmol) precursor **2b** (2-{2-[2-({5-[(E)-2-{4-[(tert-butoxycarbonyl)(methyl)amino]-phenyl}vinyl]pyridin-2-yl}oxy)ethoxy]ethoxy}ethyl 4-methylbenzenesulfonate) in 2 mL DMSO was reacted with [F-18]fluoride/kryptofix/potassium carbonate complex. The intermediate was deprotected with HCl and diluted with 1% NaOH solution for neutralization. The mixture was loaded onto a reverse phase cartridge. The cartridge was washed with water (containing 5% w/v sodium ascorbate). The crude product was eluted with acetonitrile into a reservoir containing water + 5% w/v sodium ascorbate and HPLC solvent. After purification by semi-preparative HPLC, the product fraction was collected into a reservoir containing water + 0.5% w/v sodium ascorbate. The solution was passed trough a C18 cartridge, the cartridge was washed with water (containing 0.5% w/v sodium ascorbate and the final product was eluted with ethanol into a vial containing 0.9% sodium chloride solution with 0.5% w/v sodium ascorbate.

### c) Y. Liu et al., Nuclear Medicine and Biology 37 (2010) 917-925

1 mg (1.63 µmol) precursor **2b** (2-{2-[2-({5-[(E)-2-{4-[(tert-butoxycarbonyl)(methyl)amino]-phenyl}vinyl]pyridin-2-yl}oxy)ethoxy]ethoxy}ethyl 4-methylbenzenesulfonate) in 1 mL DMSO was reacted with [F-18]fluoride/kryptofix/potassium carbonate complex (synthesis using tetrabutylammonium [F-18]fluoride in acetonitrile was found to be inferior). The intermediate was deprotected with HCl and diluted with 1% NaOH solution. The mixture was loaded onto a Oasis HLB cartridge. The cartridge was washed with water, dried under a flow of argon and the product was eluted with ethanol into a vial containing a saline solution. Although, radiochemical impurities were removed by this procedure, non-radioactive by-products derived from hydrolysis of the excess of precursor, remained in the final product solution.

The yield for 4-[(E)-2-(6-{2-[2-(2-[F-18]fluoroethoxy)ethoxy]ethoxy}pyridin-3-yl)vinyl]-N-methylaniline was 34% (non-decay corrected) within 50 min at a radioactive level from 10-100 mCi (370-3700 MBq).

A "GMP compliant" manufacturing process for 4-[(E)-2-(6-{2-[2-(2-[F-18]fluoroethoxy)ethoxy]-ethoxy}pyridin-3-yl)vinyl]-N-methylaniline is disclosed in WO2010078370 and C.-H. Yao et al., Applied Radiation and Isotopes 68 (2010) 2293-2297. The radiolabeling of 1.63 µmol-2.45 mmol was performed in DMSO and to prevent the decomposition of 4-[(E)-2-(6-{2-[2-(2-[F-18]fluoroethoxy)ethoxy]-ethoxy}pyridin-3-yl)vinyl]-N-methylaniline, sodium ascorbate was added to the HPLC solvent (45% acetonitrile, 55% 20 mM ammoniumacetate containing 0.5% (w/v) sodium ascorbate) and the final Formulation (0.5% (w/v) sodium ascorbate). The process afforded up to 18.5 GBq (25.4 ± 7.7%, decay corrected) 4-[(E)-2-(6-{2-[2-(2-[F-18]fluoroethoxy)ethoxy]-ethoxy}pyridin-3-yl)vinyl]-N-methylaniline. The radiochemical purity was 95.3 ± 2.2%.

So far, the maximum activity for a F-18 labeled fluoropegylated (aryl/heteraryl vinyl)-phenyl methyl amine derivative was reported to be 18.5 GBq (Yao et al.). However, even higher yields would be supportive for a widespread use and availability of the radiotracer. The Method of the present invention provides high yield of the F-18 tracer within a broad range of radioactivity in contrast to previous processes, wherein up-scaling is limited.

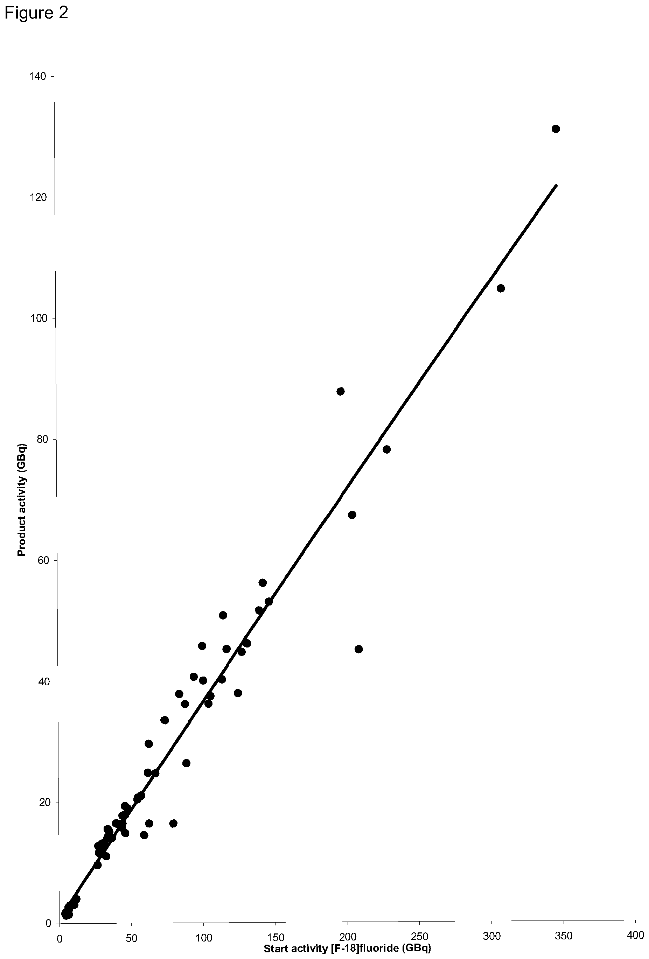

Despite the information from the literature, that amounts of less than 7.5 µmol and recently less than 12 µmol precursor are sufficient or even optimal for the preparation of F-18 labeled fluoropegylated (aryl/heteraryl vinyl)-phenyl methyl amine derivatives, a significant increase of radiochemical yield was found using more than 10 µmol or even more than 12 µmol precursor. The maximum activity for a F-18 labeled fluoropegylated (aryl/heteraryl vinyl)-phenyl methyl amine derivative was increased to 130 GBq and up-scaling was found to be almost linear (see Figure 2), demonstrating that even higher amounts of the radiotracer could be synthesized by the Method of the present invention.

### SUMMARY OF THE INVENTION

- The present invention provides a method for production of radiolabeled compound of Formula **I** according to the claims and suitable salts of an inorganic or organic acid thereof, hydrates and solvates thereof and optionally a pharmaceutically acceptable carrier, diluent, adjuvant or excipient.
   The method comprises the steps of:
   - Radiofluorination of compound of Formula **II**
   - Optionally, cleavage of the protecting group
   - Purification and formulation of compound of Formula **I** 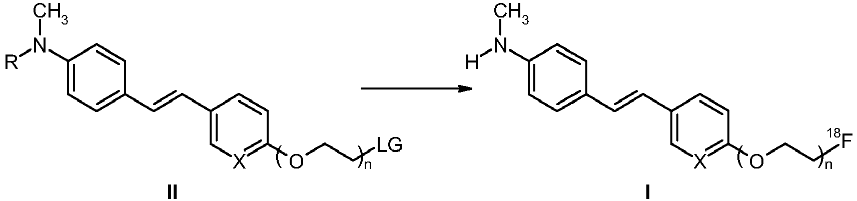
- Also disclosed herein are compositions comprising a radiolabeled compound of Formula **I** or suitable salts of an inorganic or organic acid thereof, hydrates and solvates thereof thereof and optionally a pharmaceutically acceptable carrier, diluent, adjuvant or excipient.
- Also disclosed herein is a kit for preparing a radiopharmaceutical preparation by the herein described process, said kit comprising a sealed vial containing a predetermined quantity of the compound of Formula **II**.

### DESCRIPTION OF THE INVENTION

In a ***first aspect*** the present invention is directed to a method for producing compound of Formula **I** 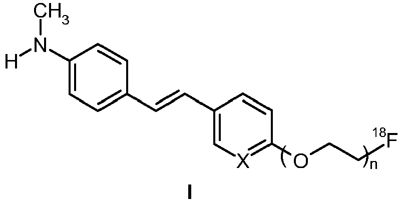 comprising the steps of:

| | |
|---|---|
| **Step 1:** | Radiolabeling by reacting compound of Formula **II** with a F-18 fluorinating agent, to obtain compound of Formula **I**, if R = H or to obtain compound of Formula **III,** if R = PG |
| 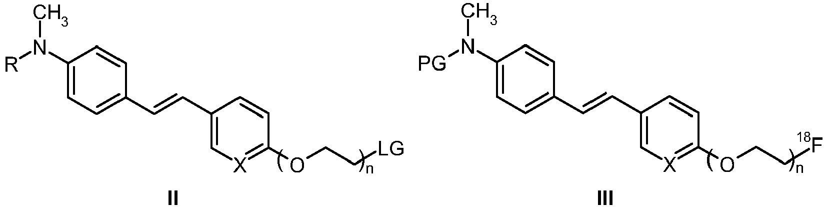 | |
| **Step 2:** | Optionally, if R = **PG,** cleavage of the protecting group **PG** to obtain compound of Formula **I** |
| **Step 3:** | Purification and formulation of compound of Formula **I** |

wherein:
**n** = 3.
**X** is selected from the group comprising
   a) CH,
   b) N.

In one preferred embodiment, **X** = CH.

In another preferred embodiment, **X** = N.

**R** is selected from the group comprising
a) H,
b) **PG.**

**PG** is an "amine-protecting group".

In a preferred embodiment, **PG** is selected from the group comprising:
a) Boc,
b) Trityl and
c) 4-Methoxytrityl.

In a more preferred embodiment, **R** is H**.**

In another more preferred embodiment, **R** is Boc.

**LG** is a leaving group selected from the group consisting of:
a) Halogen and
b) Sulfonyloxy,
wherein LG contains 0-3 fluorine atoms. Halogen is chloro, bromo or iodo.

Preferably, Halogen is bromo or chloro.

In a preferred embodiment Sulfonyloxy is selected from the group consisting of Methanesulfonyloxy, *p*-Toluenesulfonyloxy, Trifluormethylsulfonyloxy, 4-Cyanophenylsulfonyloxy, 4-Bromophenylsulfonyloxy, 4-Nitrophenylsulfonyloxy, 2-Nitrophenylsulfonyloxy, 4-lsopropyl-phenylsulfonyloxy, 2,4,6-Triisopropyl-phenylsulfonyloxy, 2,4,6-Trimethylphenylsulfonyloxy, 4-*tert*-*B*utyl-phenylsulfonyloxy, 4-Adamantylphenylsulfonyloxy and 4-Methoxyphenylsulfonyloxy.

In a more preferred embodiment, Sulfonyloxy is selected from the group comprising:
a) Methanesulfonyloxy,
b) p-Toluenesulfonyloxy
c) (4-Nitrophenyl)sulfonyloxy,
d) (4-Bromophenyl)sulfonyloxy.

In a even more preferred embodiment **LG** is Methanesulfonyloxy.

In another even more preferred embodiment **LG** is *p*-Toluenesulfonyloxy.

A preferred compound of Formula **I** is: 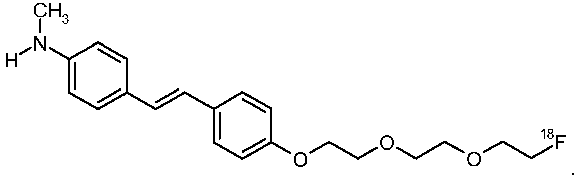 4-[(E)-2-(4-{2-[2-(2-[F-18]fluoroethoxy)ethoxy]ethoxy}phenyl)vinyl]-N-methylaniline.

Another preferred compound of Formula **I** is: 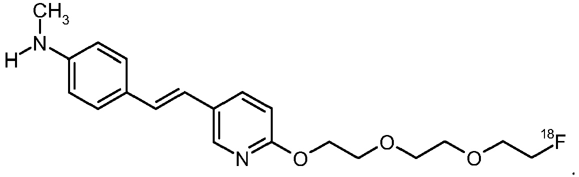 4-[(E)-2-(6-{2-[2-(2-[F-18]fluoroethoxy)ethoxy]ethoxy}pyridin-3-yl)vinyl]-N-methylaniline.

A preferred compound of Formula **II** is: 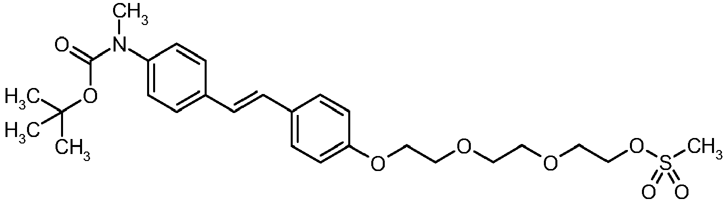 2-[2-(2-{4-[(E)-2-{4-[(*tert*-butoxycarbonyl)(methyl)amino]phenyl}vinyl]phenoxy}-ethoxy)ethoxy]ethyl methanesulfonate.

Another preferred compound of Formula **II** is: 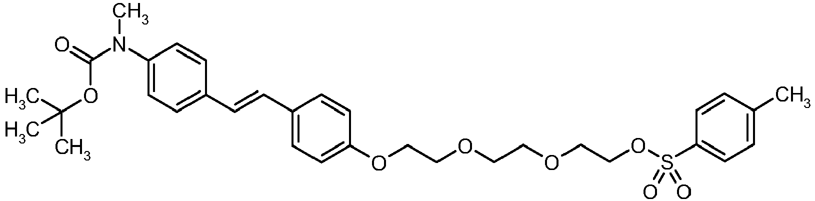 2-[2-(2-{4-[(*E*)-2-{4-[(*tert*-butoxycarbonyl)(methyl)amino]phenyl}vinyl]phenoxy}-ethoxy)ethoxy]ethyl 4-methylbenzenesulfonate

Another preferred compound of Formula **II** is: 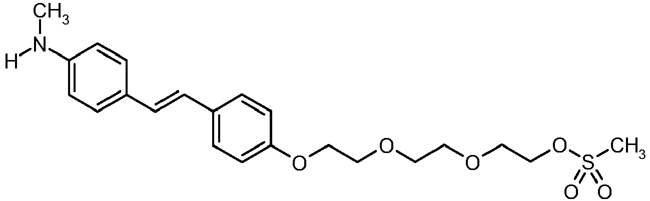 2-{2-[2-(4-{(*E*)-2-[4-(methylamino)phenyl]vinyl}phenoxy)ethoxy]ethoxy}ethyl 4-methylbenzenesulfonate

Another preferred compound of Formula **II** is: 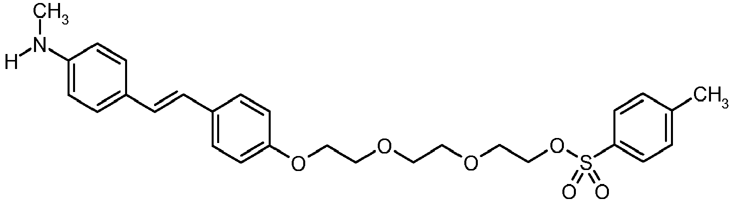 2-{2-[2-(4-{(*E*)-2-[4-(methylamino)phenyl]vinyl}phenoxy)ethoxy]ethoxy}ethyl 4-methylbenzenesulfonate

Another preferred compound of Formula **II** is: 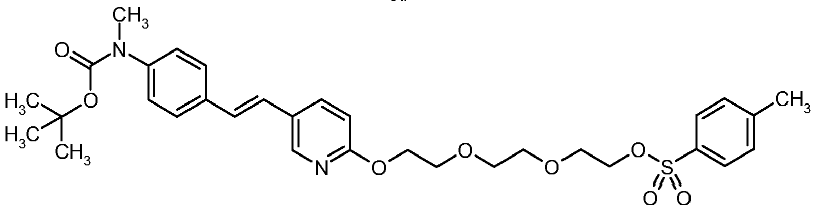 2-{2-[2-({5-[(*E*)-2-{4-[(*tert*-butoxycarbonyl)(methyl)amino]phenyl}vinyl]pyridin-2-yl}oxy)ethoxy]ethoxy}ethyl 4-methylbenzenesulfonate
**Step 1** comprises a straight forward [F-18]fluoro labeling reaction from compounds of Formula **II** for obtaining compound of Formula **I** (if R = H) or compound of Formula **III** (if R = PG).

The radiolabeling method comprises the step of reacting a compound of formula **II** with a F-18 fluorinating agent for obtaining a compound of formula **III**. In a preferred embodiment, the [F-18]fluoride derivative is 4,7,13,16,21,24-Hexaoxa-1,10-diazabicyclo[8.8.8]-hexacosane K[F-18]F (crownether salt Kryptofix K[F-18]F), K[F-18]F, H[F-18]F, KH[F-18]F₂, Cs[F-18]F, Na[F-18]F or tetraalkylammonium salt of [F-18]F (e.g.[F-18]tetrabutylammonium fluoride). More preferably, the fluorination agent is K[F-18]F, H[F-18]F, [F-18]tetrabutylammonium fluoride, Cs[F-18]F or KH[F-18]F₂, most preferably K[F-18], Cs[F-18]F or [F-18]tetrabutylammonium fluoride. An even more preferred F-18 fluorinating agent is kryptofix/potassium[F-18]fluoride, preferably generated from [F-18]fluoride, kryptofix and potassium carbonate.

The radiofluorination reactions are carried out in acetonitrile, dimethylsulfoxide or dimethylformamide or a mixture thereof. The radiofluorination reactions are conducted for less than 60 minutes. Preferred reaction times are less than 30 minutes. Further preferred reaction times are less than 15 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50).

In a preferred embodiment, the Radiofluorination of compound of Formula **II** is carried out in acetonitrile or in a mixture of acetonitrile and co-solvents, wherein the percentage of acetonitrile is at least 50%, more preferably at least 70%, even more preferably at least 90%.
10-50 µmol, more preferably 10-30 µmol and even more preferably 12-25 µmol and even more preferably 13-25 µmol of compound of Formula **II** are used in **Step 1.**

Optionally, if R = PG, **Step 2** comprises the deprotection of compound of formula **III** to obtain compound of formula **I**. Reaction conditions are known or obvious to someone skilled in the art, which are chosen from those described in the textbook Greene and Wuts, Protecting groups in Organic Synthesis, third edition, page 494-653.
Preferred reaction conditions are addition of an acid and stirring at 0 °C-180 °C; addition of an base and heating at 0 °C-180 °C; or a combination thereof. Preferably the **step 1** and **step 2** are performed in the same reaction vessel.

**Step 3** comprises the purification and formulation of compound of Formula **I**.

Methods for purification of radiotracers are well known to person skilled in the art and include HPLC methods as well as solid-phase extraction methods.

In one embodiment, the crude product mixture is purified by HPLC and the collected product fraction is further passed through a solid-phase cartridge to remove the HPLC solvent (such as acetonitrile) and to provide the compound of Formula **I** in an injectable Formulation.

In an other embodiment, the crude product mixture is purified by HPLC, wherein, the HPLC solvent mixture (e.g. mixtures of ethanol and aqueous buffers) can be part of the injectable Formulation of compound of Formula **I**. The collected product fraction can be diluted or mixed with other parts of the Formulation.

In an other embodiment, the crude product mixture is purified by solid-phase cartridges.

In a preferred embodiment, the Method for manufacturing of compound of Formula **I** is carried out by use of a module (review: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated synthesis. More preferably, the Method is carried out by use of an one-pot module. Even more preferable, the Method is carried out on commonly known non-cassette type modules (e.g. Ecker&Ziegler Modular-Lab, GE Tracerlab FX, Raytest SynChrom) and cassette type modules (e.g. GE Tracerlab MX, GE Fastlab, IBA Synthera, Eckert&Ziegler Modular-Lab PharmTracer), optionally, further equipment such as HPLC or dispensing devices are attached to the said modules.

Also disclosed herein is a fully automated and/or remote controlled Method for production of compound of Formula **I** wherein compounds of Formula **I, II** and **III** and **Steps 1, 2** and **3** are described above.

This method may be a fully automated process, compliant with GMP guidelines, that provides a Formulation of Formula **I** for the use of administration (injection) into human.

Also disclosed herein is a Kit for the production of a pharmaceutical composition of compound of Formula **I**.

The Kit may comprise a sealed vial containing a predetermined quantity of the compound of Formula **II**. The Kit may contain 10-50 µmol, more preferably 10-30 µmol and even more preferably 12-25 µmol and even more preferably 13-25 µmol of compound of Formula **II.**

Optionally, the Kit contains further components for manufacturing of compound of Formula **I**, such as solid-phase extraction cartridges, reagent for fluorination (as described above), acetonitrile or acetonitrile and a co-solvent, reagent for cleavage of deprotection group, solvent or solvent mixtures for purification, solvents and excipient for formulation.

The Kit may contain a platform (e.g. cassette) for a "cassette-type module" (such as Tracerlab MX or IBA Synthera).

### DEFINITIONS

In the context of the present invention, preferred salts are pharmaceutically suitable salts of the compounds according to the invention. The invention also comprises salts which for their part are not suitable for pharmaceutical applications, but which can be used, for example, for isolating or purifying the compounds according to the invention.

Pharmaceutically suitable salts of the compounds according to the invention include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalene disulphonic acid, acetic acid, trifluoroacetic acid, propionic acid, lactic acid, tartaric acid, malic acid, citric acid, fumaric acid, maleic acid and benzoic acid.

Pharmaceutically suitable salts of the compounds according to the invention also include salts of customary bases, such as alkali metal salts (for example sodium salts and potassium salts), alkaline earth metal salts (for example calcium salts and magnesium salts) and ammonium salts, derived from ammonia or organic amines having 1 to 16 carbon atoms, such as ethylamine, diethylamine, triethylamine, ethyldiisopropylamine, monoethanolamine, dietha-nolamine, triethanolamine, dicyclohexylamine, dimethylaminoethanol, procaine, diben-zylamine, N methylmorpholine, arginine, lysine, ethylenediamine and N methylpiperidine.

The term halogen or halo refers to Cl, Br, F or I.

The term Sulfonyloxy refers to
-O-S(O)₂-Q wherein Q is optionally substituted aryl or optionally substituted alkyl.

The term "alkyl" as employed herein by itself or as part of another group refers to a C₁-C₁₀ straight chain or branched alkyl group such as, for example methyl, ethyl, propyl, isopropyl, butyl, isobutyl, *tert*-butyl, pentyl, isopentyl, neopentyl, heptyl, hexyl, decyl or adamantyl. Preferably, alkyl is C₁-C₆ straight chain or branched alkyl or C₇-C₁₀ straight chain or branched alkyl. Lower alkyl is a C₁-C₆ straight chain or branched alkyl.

The term "aryl" as employed herein by itself or as part of another group refers to monocyclic or bicyclic aromatic groups containing from 6 to 10 carbons in the ring portion, such as phenyl, naphthyl or tetrahydronaphthyl.

Whenever the term "substituted" is used, it is meant to indicate that one or more hydrogens on the atom indicated in the expression using "substituted" is / are replaced by one ore multiple moieties from the group comprising halogen, nitro, cyano, trifluoromethyl, alkyl and O-alkyl, provided that the regular valency of the respective atom is not exceeded, and that the substitution results in a chemically stable compound, i. e. a compound that is sufficiently robust to survive isolation to a useful degree of purity from a reaction mixture.

The term "amine-protecting group" as employed herein by itself or as part of another group is known or obvious to someone skilled in the art, which is chosen from a class of protecting groups namely carbamates, amides, imides, N-alkyl amines, N-aryl amines, imines, enamines, boranes, N-P protecting groups, N-sulfenyl, N-sulfonyl and N-silyl, and which is chosen from but not limited to those described in the textbook Greene and Wuts, Protecting groups in Organic Synthesis, third edition, page 494-653.

The amine-protecting group is preferably Carbobenzyloxy (Cbz), p-Methoxybenzyl carbonyl (Moz or MeOZ), *tert*-Butyloxycarbonyl (BOC), 9-Fluorenylmethyloxycarbonyl (FMOC), Benzyl (Bn), *p*-Methoxybenzyl (PMB), 3,4-Dimethoxybenzyl (DMPM), p-methoxyphenyl (PMP) or the protected amino group is a 1,3-dioxo-1,3-dihydro-2H-isoindol-2-yl (phthalimido) or an azido group.

The term "leaving group" as employed herein by itself or as part of another group is known or obvious to someone skilled in the art, and means that an atom or group of atoms is detachable from a chemical substance by a nucleophilic agent. Examples are given e.g. in Synthesis (1982), p. 85-125, table 2 (p. 86; (the last entry of this table 2 needs to be corrected: "n-C₄F₉S(O)₂-O-nonaflat" instead of "n-C₄H₉S(O)₂-O- nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50, explicitly: scheme 4 pp. 25, scheme 5 pp 28, table 4 pp 30, Fig 7 pp 33).

Unless otherwise specified, when referring to the compounds of formula the present invention per se as well as to any pharmaceutical composition thereof the present invention includes all of the hydrates and salts.

The term "F-18" means fluorine isotope ¹⁸F. The term"F-19" means fluorine isotope ¹⁹F.

### EXAMPLES

### Determination of Radiochemical and chemical purity

Radiochemical and chemical purities of 4-[(E)-2-(4-{2-[2-(2-[F-18]fluoroethoxy)ethoxy]-ethoxy}phenyl)vinyl]-N-methylaniline and 4-[(E)-2-(4-{2-[2-(2-[F-18]fluoroethoxy)ethoxy]-ethoxy}phenyl)vinyl]-N-methylaniline were determined by analytical HPLC (column: Atlantis T3; 150x4.6 mm, 3 µm, Waters; solvent A: 5 mM K₂HPO₄ pH 2.2; solvent B: acetonitrile; flow: 2 mL/min, gradient: 0:00 min 40% B, 0:00-05:50 min 40-90% B, 05:50-05:60 min 90-40% B, 05:60-09:00 min 40% B).
- Retention time of 4-[(E)-2-(4-{2-[2-(2-[F-18]fluoroethoxy)ethoxy]-ethoxy}phenyl)-vinyl]-N-methylaniline: 3.50-3.95 min depending, on the HPLC system used for quality control. Due to different equipment (e.g tubing) a difference in retention time is observed between the different HPLC systems. The identity of 4-[(E)-2-(4-{2-[2-(2-[F-18]fluoroethoxy)ethoxy]-ethoxy}phenyl)vinyl]-N-methylaniline was proofed by co-injection with the non-radioactive reference 4-[(E)-2-(4-{2-[2-(2-[F-19]fluoroethoxy)ethoxy]-ethoxy}phenyl)vinyl]-N-methylaniline.
- Retention time of 4-[(E)-2-(6-{2-[2-(2-[F-18]fluoroethoxy)ethoxy]-ethoxy}pyridin-3-yl)vinyl]-N-methylaniline: 3.47 min. The identity of 4-[(E)-2-(6-{2-[2-(2-[F-18]fluoroethoxy)ethoxy]-ethoxy}pyridin-3-yl)vinyl]-N-methylaniline was proofed by co-elution with the non-radioactive reference -[(E)-2-(6-{2-[2-(2-[F-19]fluoroethoxy)ethoxy]-ethoxy}pyridin-3-yl)vinyl]-N-methylaniline.

### Example 1 Comparison of 4-[(E)-2-(4-{2-[2-(2-[F-18]fluoroethoxy)ethoxy]-ethoxy}phenyl)vinyl]-N-methylaniline radiosynthesis on GE Tracerlab FX_{N} using 3.5 mg vs. 7 mg mesylate precursor 2a

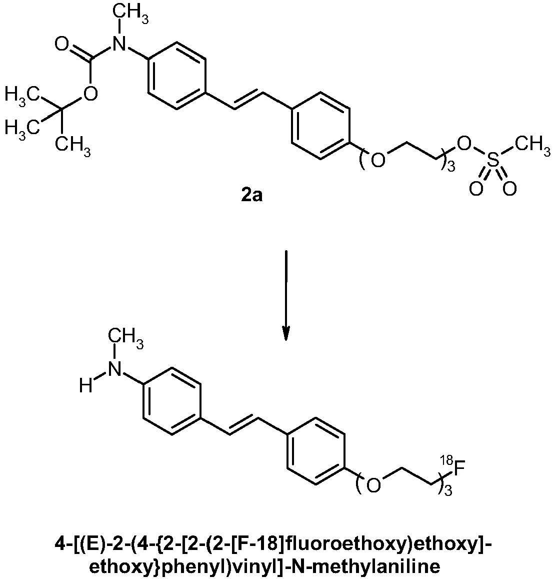

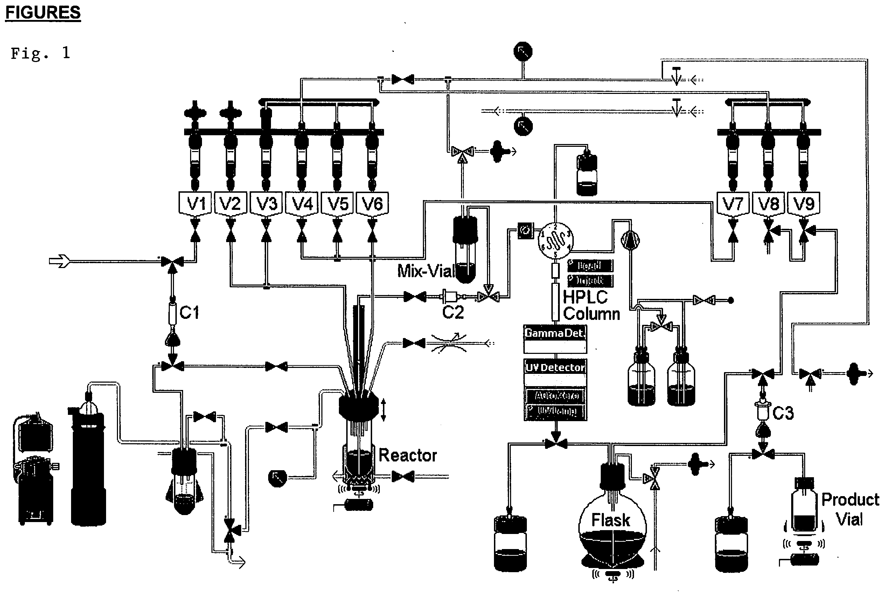

The synthesis of 4-[(E)-2-(4-{2-[2-(2-[F-18]fluoroethoxy)ethoxy]-ethoxy}phenyl)vinyl]-N-methylaniline have been performed on a Tracerlab FX_{N} synthesizer (Figure 1).

The setup of the synthesizer and the results are summarized in Table 1. [F-18]Fluoride was trapped on a QMA cartridge (C1, Figure 1). The activity was eluted with potassium carbonate/kryptofix mixture (from "V1") into the reactor. The solvent was removed while heating under gentle nitrogen stream and vacuum. Drying was repeated after addition of acetonitrile (from "V2"). The solution of **2a** (from "V3") was added to the dried residue and the mixture was heated for 8 min at 120 °C. After cooling to 60 °C, HCl/acetonitrile mixture (from "V4") was added and solution was heated for 4 min at 110 °C.

The crude product was transferred to the "Mix-Vial" and diluted with sodium hydroxide/ammonium formate mixture from "V6". The crude product was purified by semi-preparative HPLC. The product fraction was collected into the "Flask" containing 30 mL water. the solution was passed through a tC18 plus cartridge (C3). The cartridge was washed with 20% ethanol in water from "V9" and 4-[(E)-2-(4-{2-[2-(2-[F-18]fluoroethoxy)ethoxy]-ethoxy}phenyl)vinyl]-N-methylaniline was eluted with 1.5 mL ethanol into the product vial containing 8.5 mL formulation basis (consisting of phosphate buffer, PEG400 and ascorbic acid).

**Table 1 Setup of Tracerlab FX_{N} for synthesis of 4-[(E)-2-(4-{2-[2-(2-[F-18]fluoroethoxy)ethoxy]-ethoxy}phenyl)vinyl]-N-methylaniline**

| | 3.5 mg precursor | 7.0 mg precursor |
|---|---|---|
| Vial V1 | 1.5 mg potassium carbonate, 5 mg kryptofix in 0.075 mL water and 1.425 mL acetonitrile | |
| Vial V2 | 1 mL acetonitrile for drying | |
| Vial V3 | 3.5 mg (6.53 µmol) precursor **2a** in 1 mL acetonitrile | 7 mg (13.1 µmol) precursor **2a** in 1 mL acetonitrile |
| Vial V4 | 0.5 mL 2M HCl and 0.5 mL acetonitrile | |
| Vial V6 | 1 mL 1M NaOH and 2 mL ammonium formate (0.1M) | |
| Vial V8 | 1.5 mL ethanol | |
| Vial V9 | 5 mL (20% ethanol in water) + 10 mg ascorbic acid | |
| Cartridge C1 | QMA light (Waters) | |
| Cartridge C3 | tC18 plus (Waters) | |
| Flask | 30 mL water + 60 mg ascorbic acid | |
| HPLC column | Zorbax Bonus RP, 9,4*250mm; 5µm; (Agilent) | |
| HPLC solvent | 55% acetonitrile, 45% ammonium formate (0.1M) | |
| HPLC flow | 4 mL/min | |
| Start activity of [F-18]fluoride | 54000 MBq | 36600 MBq |
| Product activity | 12600 MBq | 18000 MBq |
| Radiochemical yield | 23% (not corrected for decay) | 49% (not corrected for decay) |

Significant increase of radiochemical yield for 4-[(E)-2-(4-{2-[2-(2-[F-18]fluoroethoxy)ethoxy]-ethoxy}phenyl)vinyl]-N-methylaniline was found after increasing the amount of precursor from 3.5 mg to 7.0 mg.

### Example 2 Comparison of 4-[(E)-2-(4-{2-[2-(2-[F-18]f)uoroethoxy)ethoxy]-ethoxy}phenyl)vinyl]-N-methylaniline radiosynthesis on Eckert & Ziegler ModularLab using 4.0 mg vs.7.4 mg mesylate precursor used with tert-amyl alcohol solvent for fluorination (comparative example)

The synthesis of 4-[(*E*)-2-(4-{2-[2-(2-fluoroethoxy)ethoxy]-ethoxy}phenyl)vinyl]-N-methylaniline has been performed on Eckert & Ziegler ModularLab synthesizer using *tert*-amyl alcohol as solvent for fluorination. The setup of the synthesizer and the results are summarized in Table 2.

[F-18]Fluoride was trapped on a QMA cartridge (C1). The activity was eluted with a kryptofix mixture (from "V1") into the reactor. The solvent was removed while heating under gentle nitrogen stream and vacuum. Drying was repeated after addition of acetonitrile (from "V2"). The solution of precursor (from "V3") was added to the dried residue and the mixture was heated for 12 min at 120 °C. The solvent of fluorination was removed under vacuum for 6 min at 120 °C. After cooling to 40 °C, HCl/acetonitrile mixture (from "V4") was added and solution was heated for 8 min at 120 °C.

The crude product mixture was diluted with 1.5 mL 2M NaOH and 0.3 mL ammonium formate (1M) from "V5" and then directly transferred to the HPLC vial ("Mix-Vial"). To avoid the precipitation and the phase separation of the mixture due to the *tert*-amyl alcohol, the "Mix-Vial" contained previously 1 mL acetonitrile and 1 mL ethanol.

The mixture was purified by semi-preparative HPLC. The product fraction was collected into the "Flask" containing 16 mL water. The solution was passed through a tC18 environmental cartridge (C2). The cartridge was washed with 20% ethanol in water from "V6" and 4-[(*E*)-2-(4-{2-[2-(2-fluoroethoxy)ethoxy]-ethoxy}phenyl)vinyl]-N-methylaniline was eluted with 1.5 mL ethanol from "V7" into the product vial containing 8.5 mL formulation basis (consisting of phosphate buffer, PEG400 and ascorbic acid).

A higher radiochemical yield of 38% (not corrected for decay) was obtained using 7.4 mg precursor compared to the process using 4.0 mg precursor that afforded 15% (not corrected for decay) 4-[(*E*)-2-(4-{2-[2-(2-[F-18]fluoroethoxy)ethoxy]-ethoxy}phenyl)vinyl]-N-methylaniline.

**Table 2**

| | 4.0 mg precursor | 7.4 mg precursor |
|---|---|---|
| Vial V1 | 22 mg kryptofix | |
| | 700 µL methanol | |
| | 10 µL tert-butyl ammonium carbonate 40% | |
| | 100 µL potassium mesylate 0.2M | |
| Vial V2 | 100 µL acetonitrile for drying | |
| Vial V3 | 4.0mg precursor in 100µL acetonitrile and 1.0mL tert-amyl alcohol | 7.4mg precursor in 140µL acetonitrile and 1.0mL tert-amyl alcohol |
| Vial V4 | 2mL HCl 1.5M | |
| | 1mL acetonitrile | |
| | 30mg sodium ascorbate | |
| Vial V5 | 1.5 mL NaOH 2.0M | |
| | 300 µL ammonium formate 1M | |
| | 500 µL ethanol | |
| Vial V6 | 8mL ethanol 20% | |
| | 80mg sodium ascorbate | |
| Vial V7 | 1.5mL ethanol | |
| Cartridge C1 | QMA light (waters) conditioned with potassium mesylate 0.2M | |
| Cartridge C2 | tC18 environmental (Waters) | |
| Mix-Vial | 1mL acetonitrile | |
| | 1mL ethanol | |
| Flask | 16mL water | |
| | 160mg sodium ascorbate | |
| HPLC column | Gemini C18, 10*250mm, 5µm, Phenomenex | |
| HPLC solvent | 60% acetonitrile, 40% phosphate buffer 50mM pH 4 | |
| HPLC flow | 3mL/min | |
| Start activity of [F-18]fluoride | 55.0 GBq | 30.4 GBq |
| Product activity | 8.4 GBq | 11.6 GBq |
| Product purity (RCP) | 97% | 99% |
| Radiochemical yield | 15% (not corrected for decay) | 38% (not corrected for decay) |

Significant increase of radiochemical yield for 4-[(*E*)-2-(4-{2-[2-(2-fluoroethoxy)ethoxy]-ethoxy}phenyl)vinyl]-N-methylaniline was found after increasing the amount of precursor from 4.0 mg to 7.4 mg.

### Example 3 Synthesis of 4-[(E)-2-(4-{2-[2-(2-[F-18]fluoroethoxy)ethoxy]-ethoxy}phenyl)vinyl]-N-methylaniline on Tracerlab FX_{N}

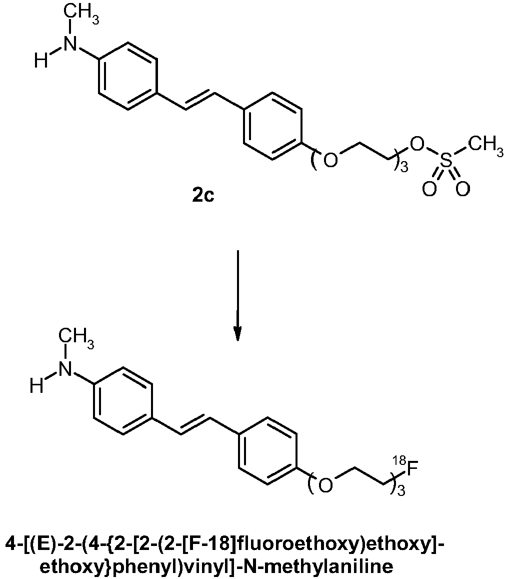

The synthesis have been performed on a Tracerlab FX_{N} synthesizer. [F-18]Fluoride (6.85 GBq) was trapped on a QMA cartridge. The activity was eluted with potassium carbonate/kryptofix/acetonitrile/water mixture into the reactor. The solvent was removed while heating under gentle nitrogen stream and vacuum. Drying was repeated after addition of acetonitrile. A solution of 8 mg **2c** in 1.5 mL acetonitrile was added to the dried residue and the mixture was heated for 10 min at 120 °C. After cooling to 60 °C, the crude product was diluted with 4 mL HPLC eluent and transferred to a semi-preparative HPLC column (Synergy Hydro-RP, 250x10mm, Phenomenex). A mixture of 60% ethanol and 40% ascorbate buffer (5g/l sodium ascorbate and 50mg/l ascorbic acid, pH 7.0) was flushed through the column with 3 mL/min. The product fraction at ≈ 12 min was directly collected for 100 sec and mixed with 15 mL Formulation basis (phosphate buffer, ascorbic acid, PEG400).
2.54 GBq (37% not corrected for decay) were obtained in 53 min overall synthesis time. Radiochemical purity (determined by HPLC, t_{R} = 3.78 min) was determined to be > 99%.

### Example 4 Up-scaling of 4-[(E)-2-(4-{2-[2-(2-[F-18]fluoroethoxy)ethoxy]-ethoxy}phenyl)vinyl]-N-methylaniline synthesis

Up-scaling of 4-[(E)-2-(4-{2-[2-(2-[F-18]fluoroethoxy)ethoxy]-ethoxy}phenyl)vinyl]-N-methylaniline synthesis was performed on two different synthesizers (Eckert & Ziegler modular lab and GE tracerlab MX) by reacting 8 mg precursor **2a** in acetonitrile at 100-120 °C for 10 min with potassium carbonate/kryptofix/[F-18]fluoride complex. The N-Boc protecting group was removed by heating with HCl (1.5M - 2M). The crude product mixture obtained after deprotection was neutralized with a mixture of 2M NaOH and 0.1M ammonium formate, diluted with acetonitrile and ethanol and injected onto a semipreparative HPLC (column: e.g.: Gemini C18, 10x250mm, 5 µm, Phenomenex or Synergi Hydro-RP, 250x10mm, 10 µm 80Å, Phenomenex or Synergi Hydro-RP, 250x10mm, 4 µm 80Å, Phenomenex; solvent: 60-70% ethanol, 40-30% ascorbate buffer ≈ 5 mg/mL ascorbate; flow 3 mL/min or 4 mL/min or 6 mL/min). The product fractions were directly collected into a vials containing "Formulation basis" (comprising PEG400, phosphate buffer and ascorbic acid) to provide 10-24 mL of the final Formulation. The peak-cutting time was adjusted in the software to obtain a Formulation comprising 15% EtOH.

The results (83 experiments) are summarized in Figure 2, wherein each dot represents a result of one individual experiment. The radiochemical purities of the radiotracer were determined by HPLC and were found to be 98.9±1.6%. The almost linear trendline indicates that similar results (radiochemical yield 37.1±5.7% not corrected for decay) are obtained within a broad range of radioactivity (product activity between 1.3 and 130.8 GBq) and that even higher product activities are obtainable by the process of the present invention.

### DESCRIPTION OF THE FIGURES

- Figure 1: Setup of Tracerlab FX_{N} (adopted from tracerlab software)
- Figure 2: Up-scaling of 4-[(E)-2-(4-{2-[2-(2-[F-18]fluoroethoxy)ethoxy]-ethoxy}phenyl)vinyl]-N-methylaniline synthesis

## Claims

1. A Method for producing a compound of Formula I 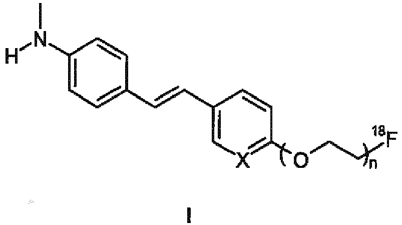 comprising the steps of:
| | |
|---|---|
| **Step 1:** | Radiolabeling compound of Formula **II** with a F-18 fluorinating agent, to obtain compound of Formula **I**, if **R =** H or to obtain compound of Formula **III**, if **R = PG** |
| | 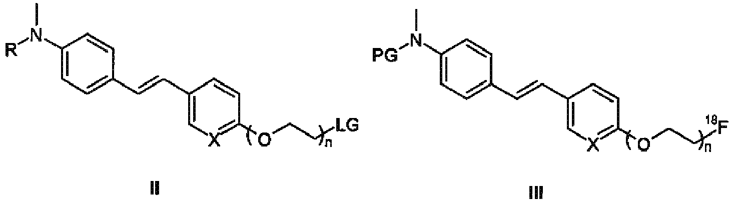 |
| **Step 2:** | If **R** = **PG**, cleavage of the protecting group **PG** to obtain a compound of Formula **I** |
| **Step 3:** | Purification and Formulation of a compound of Formula **I** |
wherein:
**n** = 3,
**X** is selected from the group consisting of
a) CH,
b) N,
**R** is selected from the group consisting of
a) H,
b) **PG**,
**PG** is an "Amine-protecting group",
**LG** is a leaving group selected from the group consisting of:
a) Halogen and
b) Sulfonyloxy,
Halogen is chloro, bromo or iodo,
wherein LG contains 0-3 fluorine atoms
wherein 10-50 µmol of a compound of formula **II** are used, wherein the radiofluorination of compound of formula **II** is carried out in acetonitrile, dimethylsulfoxide or dimethylformamide or a mixture thereof.

2. A method according to claim 1, wherein **PG** is selected from the group consisting of:
a) Boc,
b) Trityl and
c) 4-Methoxytrityl.

3. A method according to claim 1, wherein Sulfonyloxy is selected from the group comprising:
a) Methanesulfonyloxy,
b) *p*-Toluenesulfonyloxy,
c) (4-Nitrophenyl)sulfonyloxy,
d) (4-Bromophenyl)sulfonyloxy.

4. A method according to claim 1, wherein X = CH.

5. A method according to claim 1, wherein X = CH, R = Boc, and LG = Methanesulfonyloxy.

6. A method according to claim 1-5, wherein 10-30 µmol of a compound of formula II is used.

## Patentansprüche

1. Verfahren zur Herstellung einer Verbindung der Formel I 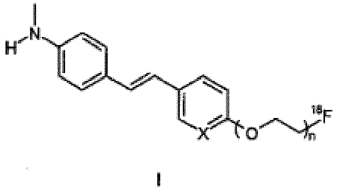 umfassend die folgenden Schritte:
| | |
|---|---|
| **Schritt 1:** | Radioaktive Markierung einer Verbindung der Formel II mit einem F-18 Fluorierungsmittel, um eine Verbindung der Formel I zu erhalten, wenn **R** = H ist, oder um eine Verbindung der Formel III zu erhalten, wenn **R** = **PG** ist |
| | 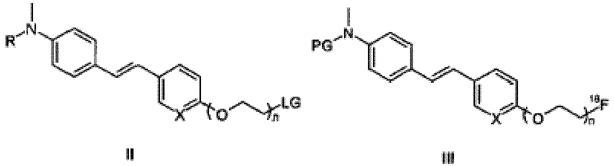 |
| **Schritt 2:** | Wenn **R** = **PG** ist, Abspaltung der Schutzgruppe **PG**, um eine Verbindung der Formel I zu erhalten |
| **Schritt 3:** | Reinigung und Formulierung einer Verbindung der Formel I |
wobei:
n = 3 ist,
**X** ausgewählt ist aus der Gruppe bestehend aus
a) CH,
b) N,
**R** ausgewählt ist aus der Gruppe bestehend aus
a) H,
b) **PG**,
**PG** eine "Amin-Schutzgruppe" ist,
**LG** eine Abgangsgruppe ist, ausgewählt aus der Gruppe bestehend aus:
a) Halogen und
b) Sulfonyloxy,
Halogen Chlor, Brom oder Iod ist,
wobei LG 0 bis 3 Fluoratome enthält
wobei 10-50 µmol einer Verbindung der Formel II verwendet werden, wobei die Radiofluorierung einer Verbindung der Formel II in Acetonitril, Dimethylsulfoxid oder Dimethylformamid oder einer Mischung aus diesen durchgeführt wird.

2. Verfahren nach Anspruch 1, wobei **PG** ausgewählt ist aus der Gruppe bestehend aus:
a) Boc,
b) Trityl und
c) 4-Methoxytrityl.

3. Verfahren nach Anspruch 1, wobei Sulfonyloxy ausgewählt ist aus der Gruppe bestehend aus:
a) Methansulfonyloxy,
b) *p*-Toluolsulfonyloxy,
c) (4-Nitrophenyl)sulfonyloxy,
d) (4-Bromphenyl)sulfonyloxy.

4. Verfahren nach Anspruch 1, wobei X = CH ist.

5. Verfahren nach Anspruch 1, wobei X = CH, R = Boc und LG = Methansulfonyloxy ist.

6. Verfahren nach den Ansprüchen 1-5, wobei 10-30 µmol einer Verbindung der Formel II verwendet werden.

## Revendications

1. Procédé pour produire un composé de la Formule I 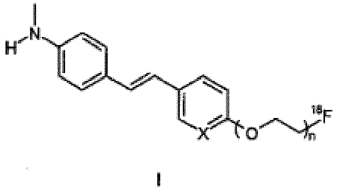 comprenant les étapes suivantes :
| | |
|---|---|
| **Étape 1** : | Radiomarquage du composé de la Formule II avec un agent de fluoration F-18, pour obtenir le composé de la formule I, si **R** = H, ou pour obtenir le composé de la formule III, si **R** = **PG** |
| | 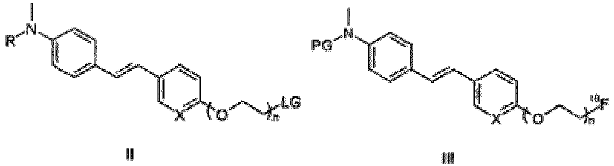 |
| **Étape 2** : | Si **R = PG,** séparation du groupe de protection PG pour obtenir le composé de la formule I |
| **Étape 3 :** | Purification et formulation d'un composé de la formule I |
dans laquelle :
**n** = 3,
**X** étant choisi parmi le groupe comprenant
a) CH,
b) N,
**R** étant choisi parmi le groupe comprenant
a) H,
b) **PG**,
**PG** étant un « groupe de protection d'amine »,
**LG** est un groupe partant choisi parmi le groupe comprenant :
a) l'halogène et
b) le sulfonyloxy,
l'halogène étant le chloro, le bromo ou l'iodo,
LG contenant 0 à 3 atomes de fluor
étant utilisés 10-50 µmol d'un composé de la formule II,
la réaction de radiofluoration d'un composé de la formule II étant effectuée dans de l'acétonitrile, le diméthylsulfoxyde ou le diméthylformamide ou un mélange de ceux-ci.

2. Procédé selon la revendication 1, **PG** étant choisi parmi le groupe comprenant :
a) le boc,
b) le trityle et
c) le 4-méthoxytrityle.

3. Procédé selon la revendication 1, le sulfonyloxy étant choisi parmi le groupe comprenant :
a) le méthanesulfonyloxy,
b) le *p*-toluènesulfonyloxy,
c) le (4-nitrophényl)sulfonyloxy,
d) le (4-bromophényl)sulfonyloxy.

4. Procédé selon la revendication 1, X = CH.

5. Procédé selon la revendication 1, X = CH, R = le boc, et LG = le méthanesulfonyloxy.

6. Procédé selon les revendications 1-5, étant utilisés 10-30 µmol d'un composé de la formule II.
